# EUROPÄISCHE PATENTSCHRIFT

(11) **EP 2 760 411 B1**
(45) Veröffentlichungstag und Bekanntmachung des Hinweises auf die Patenterteilung: **06.12.2017**
(21) Anmeldenummer: 12788417.9
(22) Anmeldetag: 11.09.2012
(51) Int. Cl.: A61H 9/00, A61F 5/01, A61F 5/34

(54) **VORRICHTUNG ZUR VERMEIDUNG VON THROMBOSEN**
DEVICE FOR PREVENTING THROMBOSIS
DISPOSITIF DE PRÉVENTION DES THROMBOSES

(30) Priorität: 28.09.2011 DE 102011114461
(43) Veröffentlichungstag der Anmeldung: 06.08.2014
(73) Patentinhaber: teveno-medi GmbH, 90522 Oberasbach (DE)
(72) Erfinder: HERRMANN, Joachim, 68782 Brühl (DE); GÜNTHER, Hans Joachim, 90403 Nürnberg (DE); THIRY, Sebastian, 8140 Bridel (LU); PANEUTZ, Willi, 90522 Oberasbach (DE)
(74) Vertreter: Küchler, Stefan
(86) Internationale Anmeldenummer: PCT/DE2012/000904
(87) Internationale Veröffentlichungsnummer: WO 2013/044894

(56) Entgegenhaltungen:
- EP-A1- 2 050 429
- WO-A2-01/74288
- WO-A2-03/068104
- US-A- 4 502 470
- US-A1- 2009 204 037
- US-A1- 2010 249 680

## Beschreibung

Die Erfindung richtet sich auf eine Vorrichtung zur Vermeidung von Thrombosen, insbesondere der tiefen Venenthrombose in den Beinvenen.

Den Beinvenen obliegt es, das vom Herzen in die Beine gepumpte Blut wieder zum Herzen zurückzuleiten. Dabei wird das Blut zunächst in feinen Ausläufern der Venen, den Venolen, gesammelt und gelangt allmählich zu Venen mit einem größeren Durchmesser. Im Bereich des Beckens wird das venöse Blut aus beiden Beinen vereinigt und strömt über die untere Hohlvene als Hauptsammelvene zum Herzen.

Das Venensystem der Beine lässt sich untergliedern in ein oberflächliches Venensystem, welches sich unmittelbar unter der Haut befindet, sowie ein tiefes Venensystem mit mehreren, tiefer im Inneren der Beine verlaufenden Venen, vorzugsweise nahe den dortigen Arterien. Zwischen dem oberflächlichen und dem tiefen Venensystem erstrecken sich zahlreiche Verbindungsvenen, welche auch als Perforansvenen bezeichnet werden.

Während die vena saphena magna als größte oberflächliche Vene des Beins entlang der Innenseite des Ober-und Unterschenkels verläuft, befindet sich die vena saphena parva als zweitgrößte oberflächliche Vene an der Außenseite des Unterschenkels.

Da im Unterschied die tiefen Beinvenen mehrfach vorhanden sind, verlaufen in den tiefen Weichteilen des Unterschenkels jeweils mehrere venae tibiales anteriores, posteriores et fibulares.

In den Venen angeordnete Venenklappen dienen als eine Art Rückschlagventil und stellen sicher, dass das Blut in alle Venen jeweils nur in einer Richtung fließen kann, nämlich in den tiefen und oberflächlichen Venen in Richtung zum Herzen hin, in den Perforansvenen dagegen nur von den oberflächlichen zu den tiefen Venen.

Dabei fließen etwa 90 Prozent des Blutes von den Füßen über das tiefe Beinvenensystem zum Herzen zurück, und zwar nicht in einem pulsierenden, vom Herzschlag angetriebenen Strom wie innerhalb der Arterien, sondern in einem kontinuierlichen Strom. Da der arterielle Blutdruck im Endbereich der Arterien nahezu vollständig aufgebraucht ist, kann dieser allenfalls in der liegenden Position eines Menschen dessen venösen Blutstrom treiben. Deshalb hat die Natur für den venösen Blutstrom weitere Antriebsmittel vorgesehen. Nur einen geringen Beitrag hierzu vermag das physiologische Druckgefälle zwischen linker und rechter Herzhälfte zu leisten. Ein weiterer, ebenfalls nur mäßiger Antriebsfaktor basiert auf der Atmung und den daraus folgenden Druckveränderungen im Körper. Hinzu kommt weiterhin die Tatsache, dass die tiefen Beinvenen zumeist in unmittelbarer Nähe der Arterien angeordnet sind und daher indirekt über den pulsierenden Blutdruck in den Arterien beeinflusst, d.h. komprimiert werden. Durch die Venenklappen wird diese Pulsation in einen herzwärts fließenden venösen Blutstrom umgesetzt. Jedoch besteht kein Zweifel daran, dass der maßgebendste Beitrag zum Antrieb des venösen Blutstroms von der sog. Wadenmuskelpumpe geliefert wird. Diese funktioniert durch abwechselnde Querschnittsveränderungen, welche die Wadenmuskulatur bei ihrer Tätigkeit erfährt: Verdickung bei angespannter Wadenmuskulatur und Reduzierung bei erschlaffendem Wadenmuskel. Während der angespannte Wadenmuskel das Blut aus den tiefen Venen drückt, und zwar - bedingt durch die Orientierung der Venenklappen - herzwärts, wird bei einer Entspannung des Wadenmuskels Blut durch die Perforansvenen aus dem oberflächlichen Venensystem angesaugt. Ähnlich wie bei einer Kaskade wird durch diesen Mechanismus das venöse Blut allmählich von den Füßen bis zum Herzen hin angehoben, wobei ein Absacken des Blutes von den Venenklappen verhindert wird.

Ein beständiger Strom des venösen Blutes ist äußerst wichtig, da nur langsam oder gar nicht strömendes Blut zur Verklumpung bzw. Bildung von Thromben neigt. Dies wiederum ist sehr gefährlich, weil derartige Thromben rasch in die Lunge gelangen und dort eine Lungenembolie auslösen können. Statistiken haben gezeigt, dass die tiefe Venenthrombose - also die Bildung von Thromben in der tiefen Beinvene - nach dem Herzinfarkt und dem Schlaganfall die dritthäufigste akut auftretende kardiovaskuläre Erkrankung ist.

Deshalb ist der Ausfall der Wadenmuskelpumpe- u.a. bei langem Sitzen wie bspw. Auf Langstreckenflügen oder unter sonstigen, beengten Verhältnissen - sehr kritisch und birgt ein großes Risiko für die betroffenen Personen.
Vielfach angebotene Stützstrümpfe können hierzu keinen ausreichenden Beitrag leisten, da die dynamische Wirkungsweise der Wadenmuskelpumpe - abwechselndes Komprimieren und Entlasten der tiefen Venen - durch eine derart statische Anordnung nicht simuliert wird.

WO 01/74288 A2 offebart eine Vorrichtung zur Vermeidung der tiefen Venenthrombose in den Beinvenen, umfassend wenigstens ein abgeschlossenes System mit zwei miteinander kommunizierenden, von einer flexiblen Hülle umgebenen, mit einem strömungsfähigen Medium befüllten Kammern, nämlich einer unterhalb einer Fußsohle anordenbaren Kompressionskammer und einer Expansionskammer, die in von äußeren Kräften freiem Zustand und bei innerem Druckausgleich zwischen beiden Kammern ein kleineres Volumen aufweist als die Kompressionskammer, wobei die Kompressionskammer sich ganz unter der Ferse befindet, die Expansionskammer sich über der Stelle befindet, an der die Vena perforantis auf der hinteren Knöchelinnenseite unter den Muskeln hervortritt, und die Expansionskammer, die Kompressionskammer und ein schlauchartiges Verbindungsstück zwischen ihnen aus folienverschweissbarem Kunststoff bestehen und aus einem Stück gefertigt sind.

Das die Erfindung initiierende Problem besteht darin, eine Vorrichtung zu schaffen, welche unter Bedingungen, die zum Ausfall der Wadenmuskelpumpe führen, wie bspw. Langes Sitzen unter beengten Verhältnissen, den venösen Blutstrom in den Beinen zusätzlich anzutreiben in der Lage ist, um ggf. ein Defizit der Wadenmuskelpumpe zu kompensieren.
Die Lösung dieses Problems gelingt durch eine Vorrichtung zur Vermeidung einer tiefen Venenthrombose in den Beinvenen mit den Merkmalen von Anspruch 1.

Die Maßnahme, gezielt auf eine oder mehrere Perforansvenen einzuwirken, hat gegenüber anderen Methoden den Vorteil, dass bei einem Pumpzyklus das Blut von der dabei ggf. ebenfalls komprimierten Oberflächenvene fast vollständig in das tiefe Venensystem gepumpt wird, von wo es nur nach oben strömen kann, weil ein Absacken durch die Venenklappen verhindert wird. Nach Entspannen der Expansionskammer kann sie die Perforansvene aufgrund der darin angeordneten Venenklappen nur mit Blut aus der Oberflächenvene füllen.
Bei mehrmaligem Wippen mit dem Fuß, wobei jedes Mal Druck auf die Kompressionskammer ausgeübt und über die Schlauchverbindung an die Kompressionskammer weitergegeben wird, kann also wiederholt Blut durch die Perforansvene in die tiefe Vene gepumpt werden, und damit ist ein verstärkter Blutfluss - gerade auch in dem von Thrombosen besonders gefährdeten tiefen Venensystem - gewährleistet, der die Bildung von Thrombosen verhindert. Die Vorrichtung umfasst ein Halteband, von welchem das Fußgelenk umschließbar ist. Wichtig dabei ist, dass das Halteband die Expansionskammer außen umschließt, und darüber hinaus zwar flexibel, also biegsam ist, aber möglichst wenig bis gar nicht elastisch ist, damit sich seine effektive Länge auch unter (Druck-) Belastung seitens der Expansionskammer nicht verändert. Ein geeignetes Material wäre bspw. Jenes, welches bei (Auto-) Sicherheitsgurten Verwendung findet.
Die effektive Länge des Haltebandes - also diejenige Länge, welche das Fußgelenk der betreffenden Person umschließt - kann einstellbar sein. Diese Verstellmöglichkeit kann auf unterschiedlichen Wegen erzielt werden, bspw. Wie bei dem Schloss eines Gurtes oder der Schnalle eines Gürtels. Die dabei erforderliche Arretierung kann mittels Formschluss erfolgen, bspw. unter Durchdringung des Haltebandes von einem Teil eines Gurtschlosses oder einer Gürtelschnalle, oder aber mittels einer Klemmung, wie bspw. bei Hosenträgern praktiziert; darüber hinaus ist auch ein Anheften denkbar, bspw. nach Art eines Klettverschlusses, wenn dieser ausreichend fest ausgebildet ist.

Das freie Ende der Kompressionskammer ist über ein kurzes Verbindungsband mit dem Halteband verbunden. Dadurch ist die präzise Lage der Kompressionskammer unterhalb der Fußsohle der betreffenden Person sowie insbesondere der geöffnete Zustand des Verbindungsteils oder - schlauchs gewährleistet.
In von äußeren Kräften freiem Zustand und bei innerem Druckausgleich zwischen beiden Kammern weist die Kompressionskammer ein größeres Volumen auf als die Expansionskammer. Dadurch kann die Expansionskammer beim Stauchen der Kompressionskammer deren Volumeninhalt aufnehmen und sich dabei entsprechend stark aufdehnen.
Dies ist besonders effektiv, wenn als Füllmedium eine inkompressible Flüssigkeit verwendet wird, wie bspw. Luft, was allerdings grundsätzlich auch möglich ist, insbesondere, wenn die obige Volumenbedingung eingehalten ist.
Bevorzugt weisen beide Kammern eine flächige Struktur auf. Damit ist für eine Volumenänderung eine Vorzugsrichtung vorgegeben, nämlich bevorzugt etwa lotrecht zu der Grundfläche. Denn dabei ist eine Volumenveränderung bei geringster Verformung der betreffenden Kammerhülle erreichbar. Deshalb können solchenfalls auch ein oder beide Kammern aus einem zwar flexiblen und damit biegsamen, aber mehr oder weniger inelastischem und damit wenig bis gar nicht dehnbarem Material bestehen, ohne dass dadurch die Funktionstüchtigkeit eingeschränkt wäre.

Bei flächig vorgeformten, flexiblen Kammerhüllen kann eine verhältnismäßig starke Volumensvergrößerung der Expansionskammer, insbesondere lotrecht zu ihrer Grundfläche, erzielt werden, wenn in von äußeren Kräften freiem Zustand und bei innerem Druckausgleich zwischen beiden Kammern die Kompressionskammer mindestens die gleiche Grundfläche aufweist, wie die Expansionskammer. Diese Bedingung kann durch eine entsprechende Vorformung erreicht werden, bspw. durch entsprechend große Zuschnitte des dichten, insbesondere luft- und/oder wasserdichten Hüllenmaterials, welche paarweise aufeinander gelegt und randseitig dicht miteinander verbunden werden, bspw. verscheißt oder verklebt.
Bevorzugt besteht die Verbindungsleitung zwischen Kompressionskammer und Expansionskammer aus einem inelastischen Material. Dadurch trägt dieser Teil der miteinander verbundenen Kammern nicht zu einer Volumensveränderung bei, vielmehr muss das gesamte, aus der Kompressionskammer verdrängte Volumen von der Expansionskammer aufgenommen werden.

Die Erfindung sieht vor, dass die Verbindungsleitung zwischen Kompressionskammer und Expansionskammer an der Innenseite des Fußes entlangläuft. Dies ist die kürzeste Verbindung zwischen der Kompressionskammer, welche sich ganz unterhalb der Ferse der betreffenden Person befindet, und der Expansionskammer zwischen Innenknöchel und Achillessehne.
Weitere Vorteile ergeben sich dadurch, dass die erfindungsgemäße Vorrichtung in einem Schuh/0der unter einem Strumpf tragbar ist. Insofern muss die Vorrichtung von außen überhaupt nicht sichtbar sein und ist also äußerst dezent. Sie kann von einer Person in besonderen Fällen angezogen werden, bspw. vor einem längeren Flug, oder aber - insbesondere Personen mit sitzendem Beruf - täglich getragen werden.
Eine weitere Konstruktionsvorschrift sieht vor, dass die Wade von der erfindungsgemäßen Vorrichtung nicht bedeckt ist. Jegliche Einengung der Wade kann auf Dauer eher einen kontraproduktiven Einfluss auf die Beindurchblutung haben und sollte daher vermieden werden.
Schließlich entspricht es der Lehre der Erfindung, dass die erfindungsgemäße Vorrichtung asymmetrisch ist. Grundsätzlich ist es möglich, die Vorrichtung derart universell zu konstruieren, dass sie durch eine 180°-Drehung um eine vertikale Achse in eine zu sich selbst spiegelbildliche Struktur übergeht und sodann für den jeweils anderen Fuß der betreffenden Person verwendbar ist. Dieses Merkmal sollte jedoch nicht im Vordergrund einer optimalen Konstruktion stehen, sondern vielmehr eine optimale Funktion. Bspw. kann der Umfang einer flächigen Kompressionskammer im rückwärtigen Bereich der Ferse einer runden Kurve folgen, im vorderen Bereich der Ferse dagegen einen anderen Verlauf aufweisen, bspw. einen gerade gestreckten.

Die Erfindung zeichnet sich ferner aus durch ein System zur Vermeidung einer Thrombose, insbesondere der tiefen Venenthrombose, in den Venen beider Füße, mit einem Paar von zueinander spiegelsymmetrischen Vorrichtungen mit einem oder mehreren der zuvor beschriebenen Merkmale, für den linken und rechten Fuß einer Person. Durch den paarweisen Einsatz kann eine thrombosegefährdete Person das Risiko einer Thrombose minimieren oder gar eliminieren.

Weitere Merkmale, Einzelheiten, Vorteile und Wirkungen auf der Basis der Erfindung ergeben sich aus der folgenden Beschreibung einer bevorzugten Ausführungsform der Erfindung sowie anhand der Zeichnung. Hierbei zeigt:
Fig.1 eine perspektivische Ansicht einer erfindungsgemäßen Vorrichtung in an einem Fuß applizieren Zustand;
Fig. 2 die Wirkungsweise der Erfindung bei belastetem und entlastetem Fuß; sowie
Fig. 3 das Kammersystem einer erfindungsgemäßen Vorrichtung, in die Zeichenebene abgewickelt.

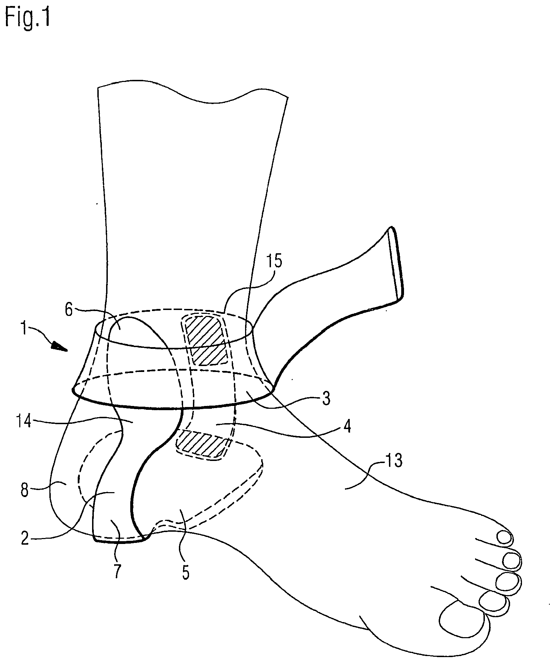

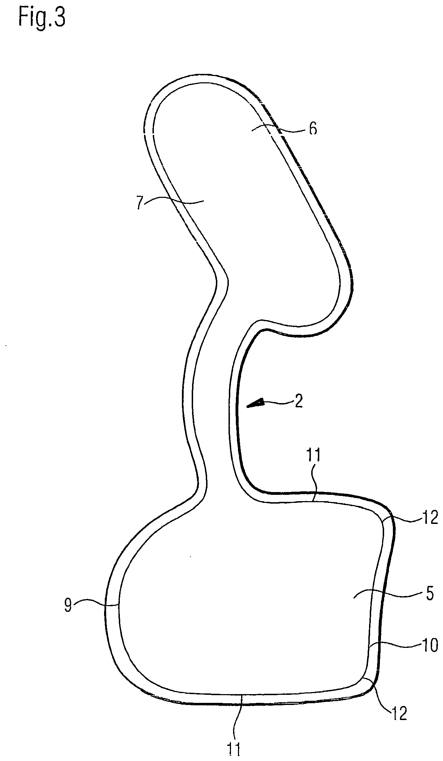

Wie man der Fig. 1 entnehmen kann, besteht eine erfindungsgemäße Vorrichtung 1 zur Vorbeugung gegenüber Beinthrombosen im Wesentlichen aus drei Teilen: Dem eigentlichen Kammersystem 2, einem in sich geschlossenen Halteband 3 und einem beide Teile 2,3 zusätzlich miteinander verbindenden Verbindungsband 4. Das Kammersystem 2 ist in Fig. 3 in einem ebenen Zustand wiedergegeben, so dass dessen dreiteiliger Aufbau erkennbar ist: Eine Kompressionskammer 5 kommuniziert mit einer Expansionskammer 6 über ein schlauchförmiges Zwischenstück 7.

Die in Fig. 3 unten dargestellte Kompressionskammer 5 ist etwa der Grundfläche einer menschlichen Ferse 8 nachempfunden weist im hinteren, in Fig. 3 linken Bereich einen konvex gewölbten, rückwärtigen Rand 9 auf entsprechend dem rückwärtigen Bereich einer Fußsohle, der vordere Rand 10 sowie die jene Randbereiche 9,10 miteinander verbindenden seitlichen Ränder 11 haben demgegenüber einen eher geraden Verlauf, der vordere Rand 10 könnte sogar einen leicht konkaven Verlauf haben; dessen Eckbereiche 12 sind abgerundet. Insgesamt kann die Kompressionskammer 5 etwa gleiche Erstreckungen in Längs- und Querrichtung aufweisen.

Die Expansionskammer 6 hat eine eher längliche Gestalt, mit einem etwa elliptischen Umfang, wobei die Länge durchaus zwischen dem Doppelten oder Dreifachen seiner Breite sein kann.
Das die beiden Kammern 5.6 miteinander verbindende, schlauchförmige Zwischenstück 7 setzt an einem Seitenbereich 11 der Kompressionskammer 5 an, vorzugsweise im hinteren Bereich derselben.
Bevorzugt folgt das Schlauchförmige Zwischenstück 7 einem leicht gebogenen Verlauf bis zu der Expansionskammer 6. Die Breite des schlauchförmigen Zwischenstücks 7 ist vorzugsweise jedoch etwa konstant.
Das schlauchförmige Zwischenstück 7 mündet an der Expansionskammer 6 an einem Ende der Längsseite 12 ihres Umfangs.
Die Längsachsen der beiden Kammern 5,6 schließen einen spitzen Winkel miteinander ein, vorzugsweise in der Größenordnung zwischen 30° und 90°, insbesondere zwischen 45° und 75°, bevorzugt etwa 60°.
Alle drei Teile 5 bis 7 können durch zwei deckungsgleiche, aufeinander gelegte Zuschnitte aus einem medizinisch verträglichen bzw. hautverträglichen Material gebildet sein, welche an ihrem gesamten Umfang dicht miteinander verschweißt sind. Das Material sollte luft- und wasserundurchlässig sein, bspw. aus Gummi ähnlich dem Material einer Wärmeflasche. Denkbar ist auch ein mehrlagiger Aufbau aus einem gas- oder flüssigkeitsdichten Material im Inneren, das von einem besonders hautverträglichen Material außen umgeben ist.

Die über den Schlauch 7 miteinander kommunizierenden Kammern 5,6 sind mit einem strömungsfähigen Medium befüllt, vorzugsweise einem flüssigen Medium, wie Wasser. Das Füllvolumen sollte derart gewählt sein, dass die Kammern 5,6 im ursprünglichen Zustand nicht prall aufgeblasen sind; bspw. könnte das Füllvolumen etwa dem maximalem Volumen der Expansionskammer 6 in deren zwar gut oder gar prall gefüllten Zustand, jedoch ohne Dehnung derselben entsprechen. Je nach Art der Befüllung und ggf. entsprechend dem Wunsch einer Möglichkeit zur Nachbefüllung kann ein Ventil vorgesehen sein, bspw. nach Art eines Fahrradventils, oder auch nicht. Anstelle von Wasser oder Luft sind auch andere Füllmedien denkbar, bspw. Stickstoff.

Wie Fig. 1 erkennen lässt, wird dieses Kammersystem 2 mittels zweier Bänder 3,4 an dem Fuß 13 einer Person im Bereich der Ferse 8 befestigt. Ein dazu verwendetes Halteband 3 kann an der Außenseite 14 der Expansionskammer 6befestigt sein, derart, bevorzugt etwa in der Mitte des Haltebandes 3. Seine beiden Enden werden um das Fußgelenk 15 geschlungen, wie dies in Fig. 1 dargestellt ist, und schließlich im Bereich der Außenseite des Fußes 13 miteinander verbunden, bspw. mit einem Klettverschluss, einem Klemmverschluss od. dgl.

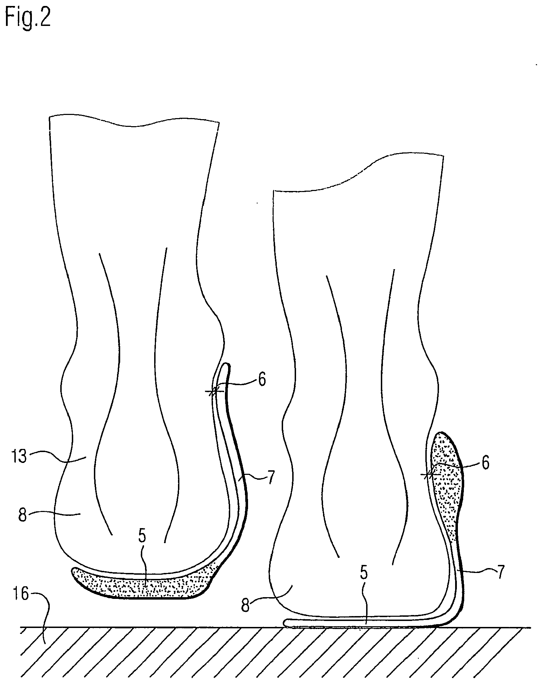

Wichtig ist, dass das Halteband 3 kaum dehnbar ist und also eng um das Fußgelenk 15 gelenkt werden kann, so dass das Füllmedium bei entlasteter Kompressionskammer 5 aus der Expansionskammer 6 herausgedrückt wird, wodurch der auf den Innenknöchelbereich des Fußes 13 ausgeübte Druck minimal wird - die Perforansvene unterhalb bzw. neben der Expansionskammer 6 saugt Blut aus dem Oberflächenvenensystem. Dieser Zustand ist in Fig. 2 links dargestellt, bspw. bei vom Boden 16 angehobenem Fuß 13. Das Überströmen des Füllmediums von der Expansionskammer 6 in die Kompressionskammer 5 kann zusätzlich unterstützt werden, indem die Kompressionskammer 5 mit einem porösen, elastisch sich aufdehnenden Material befüllt wird, bspw. mit einem Schwamm, der unter Druck bestrebt ist, sich auszudehnen und dabei das Füllmedium in die Kompressionskammer 5 saugt.

Wird der Fuß 13 anschließend auf den Boden 16 gestellt oder gar gepresst, so strömt dabei das Füllmedium von der Kompressionskammer 5 in die Expansionskammer 6 im Bereich des Fuß-Innenknöchels, wie in Fig. 2 rechts dargestellt. Weil die dabei befüllte und sich aufblähende Expansionskammer 6 aufgrund des dieselbe außen umschließenden Haltebandes 3 nicht nach außen ausweichen kann, drückt sie verstärkt gegen den Bereich des Fuß-Innenknöchels - die Perforansvene unterhalb bzw. neben der Expansionskammer 6 wird komprimiert und dabei entleert, und zwar aufgrund der Venenklappen in das tiefe Venensystem. Von dort wird das Blut schließlich aufgrund des bei mehrfachen Fußbewegungen allmählich steigenden Drucks in der tiefen Vene aktiv nach oben, also herzwärts gepresst.

Durch kleine auf und ab wippende Bewegungen mit dem Fuß kann eine Person also den venösen Blutfluss in den Beinen aktiv unterstützen. Das Verbindungsband 4 dient dabei dazu, die Kompressionskammer 5 beständig an der vorgesehenen Position genau unterhalb der Ferse 8 zu halten.

Die Erfindung umfasst nicht nur die zuvor beschriebene, bandageförmige Vorrichtung als solche, sondern dieselbe kann darüber hinaus auch mit einer Einlegesohle, einem Strumpf, einer Sandale oder einem Schuh integriert werden, oder aber einzeln, d.h., als in sich abgeschlossenes Kammersystem, verwendet werden.

### Bezugszeichenliste

- 1: Vorrichtung
- 2: Kammersystem
- 3: Halteband
- 4: Verbindungsband
- 5: Kompressionskammer
- 6: Expansionskammer
- 7: Zwischenstück
- 8: Ferse
- 9: Rückwärtiger Rand
- 10: Vorderer Rand
- 11: Seitlicher Rand
- 12: Längsseite
- 13: Fuß
- 14: Außenseite
- 15: Fußgelenk
- 16: Boden

## Patentansprüche

1. Vorrichtung (1) zur Vermeidung der tiefen Venenthrombose in den Beinvenen, umfassend wenigstens ein abgeschlossenes System (2) mit zwei miteinander kommunizierenden, von einer flexiblen Hülle umgebenen, mit einem strömungsfähigen Medium befüllten Kammern, nämlich einer unterhalb einer Fußsohle anordenbaren Kompressionskammer (5) und einer Expansionskammer (6), die in von äußeren Kräften freiem Zustand und bei innerem Druckausgleich zwischen beiden Kammern (5,6) ein kleineres Volumen aufweist als die Kompressionskammer (5), wobei
a) die Kompressionskammer (5) sich bei Benutzung ganz unter der Ferse befindet;
b) die Expansionskammer (6) eine längliche Gestalt aufweist mit einem etwa elliptischen Umfang, und zwischen dem Innenknöchel und der Achillessehne anordenbar ist, in unmittelbarem Kontakt mit einem dortigen Perforansvenensystem, so dass bei einer Komprimierung der an der Fußsohle anordenbaren Kompressionskammer (5) von der dann expandierenden Expansionskammer (6) das Blut aus einer dortigen Perforansvene in eine tiefe Vene pressbar ist;
c) ein die Kompressionskammer (5) und die Expansionskammer (6) miteinander verbindendes, schlauchförmiges Zwischenstück (7) bei Benutzung an der Innenseite des Fußes entlangläuft;
d) die Vorrichtung ein flexibles aber nicht elastisches oder kaum dehnbares Halteband (3) umfasst, welches die Expansionskammer außen umschließt und von welchem das Fußgelenk derart umschließbar ist,
e) dass die sich bei ihrer Befüllung aus der Kompressionskammer (5) aufblähende Expansionskammer (6) nicht nach außen ausweichen kann, sondern die Expansion der Expansionskammer (6) an die Perforansvene unterhalb der Expansionskammer (6) weitergegeben wird, welche dabei komprimiert und entleert wird, und wobei
f) die Vorrichtung (1) bei Benutzung die Wade nicht bedeckt,
g) wobei ferner die Kompressionskammer (5), die Expansionskammer (6) sowie das schlauchförmige Zwischenstück (7) durch zwei deckungsgleiche, aufeinander gelegte Zuschnitte aus einem medizinisch verträglichen Material gebildet sind, welche an ihrem gesamten Umfang dicht miteinander verschweißt sind,
h) und wobei schließlich ein freier Seitenbereich (11) der Kompressionskammer (5) über ein kurzes Verbindungsband (4) mit dem Halteband (3) verbunden ist.

2. Vorrichtung (1) nach Anspruch 1, **dadurch gekennzeichnet, dass** die effektive Länge des Haltebandes (3) einstellbar ist.

3. Vorrichtung (1) nach einem der vorhergehenden Ansprüche, **dadurch gekennzeichnet, dass** in von äußeren Kräften freiem Zustand und bei innerem Druckausgleich zwischen beiden Kammern (5,6) die Kompressionskammer (5) das doppelte Volumen der Expansionskammer (6) aufweist oder mehr, insbesondere das dreifache Volumen der Expansionskammer (6) oder mehr.

4. Vorrichtung (1) nach einem der vorhergehenden Ansprüche, **dadurch gekennzeichnet, dass** beide Kammern (5,6) eine flächige Struktur aufweisen.

5. Vorrichtung (1) nach Anspruch 4, **dadurch gekennzeichnet, dass** in von äußeren Kräften freiem Zustand und bei innerem Druckausgleich zwischen beiden Kammern (5,6) die Kompressionskammer (5) die doppelte Grundfläche der Expansionskammer (6) aufweist oder mehr, insbesondere die dreifache Grundfläche der Expansionskammer (6) oder mehr.

6. Vorrichtung (1) nach einem der vorhergehenden Ansprüche, **dadurch gekennzeichnet, dass** sie in einem Schuh und/oder unter einem Strumpf tragbar ist.

7. Vorrichtung (1) nach einem der vorhergehenden Ansprüche, **dadurch gekennzeichnet, dass** sie asymmetrisch ist.

8. System zur Vermeidung der tiefen Venenthrombose in den Venen beider Beine, **gekennzeichnet durch** ein Paar von zueinander spiegelsymmetrischen Vorrichtungen (1) nach einem der vorhergehenden Ansprüche für den linken und rechten Fuß (13) einer Person.

## Claims

1. Device (1) for avoiding deep vein thrombosis in the leg veins, comprising at least one closed system (2) with two chambers communicating with each other and filled with a fluid medium surrounded by a flexible covering, namely one compression chamber (5) located underneath a foot sole and one expansion chamber (6) which has a smaller volume than the compression chamber (5) in a condition free from external forces and in case of internal pressure equalization between the two chambers (5, 6), wherein
a) In use, the compression chamber (5) is located entirely underneath the heel;
b) the expansion chamber (6) has a longitudinal shape with an approximately elliptic perimeter, and is located between the inner ankle and the achilles tendon, in close contact with a local perforating vein system so that, when the compression chamber (5) arranged on the foot sole is compressed, the blood is pressed by the in that case expanded expansion chamber (6) from a local perforating vein into a deeper vein;
c) in use, a tubular connecting element (7) connecting the compression chamber (5) and the expansion chamber (6) extends along the inner side of the foot;
d) the device comprises a retaining band (3) which is flexible, but not elastic or hard to expand, and which encloses the expansion chamber from outside and by which the foot ankle can be enclosed in such that
e) the expansion chamber (6) which is expanding after being filled from the compression chamber (5) cannot evade outwardly, but that the expansion of the expansion chamber (6) is transferred to the perforation vein underneath the expansion chamber (6) thereby compressing it and discharging it, and wherein
f) the calf is not covered by the device (1),
g) further wherein the compression chamber (5), the expansion chamber (6) as well as the tubular connecting element (7) are formed by two congruent cuttings of a medically tolerable material which are put on each other, and which are completely welded in a close manner along their entire perimeter,
h) and finally wherein a free side section (11) of the compression chamber (5) is connected to the retaining band (3) via a short connecting band (4).

2. Device (1) according to claim 1, **characterized in that** the effective length of the retaining band (3) is adjustable.

3. Device (1) according to one of the preceding claims, charcterized in that, in a condition free of external forces and in case of an internal pressure equalization between the two chambers (5, 6), the compression chamber (5) has double the volume of the expansion chamber (6) or more, in particular the triple volume of the expansion chamber (6) or more.

4. Device (1) according to one of the preceding claims, **characterized in that** that both chambers (5, 6) have a two-dimensional structure.

5. Device (1) according to claim 4, **characterized in that**, in a condition free of external forces and in case of an internal pressure equalization between the two chambers (5, 6), the compression chamber (5) has double the base area of the expansion chamber (6) or more, in particular the triple base area of the expansion chamber (6) or more.

6. Device (1) according to one of the preceding claims, **characterized in that** it can be worn in a shoe and/or under a stocking.

7. Device (1) according to one of the preceding claims, **characterized in that** it is asymmetrical.

8. System for avoiding thrombosis in the deep veins of both legs, **characterized by** a pair of mutually mirror-symmetrical devices (1) according to one of the preceding claims for the left and right foot (13) of a person.

## Revendications

1. Dispositif (1) de prévention de la thrombose veineuse profonde des veines de la jambe, comprenant au moins un système fermé (2) comportant deux chambres communiquant l'une avec l'autre, entourées d'une gaine flexible et remplies d'un fluide apte à l'écoulement, à savoir une chambre à compression (5) pouvant être disposée sous une plante du pied et une chambre à expansion (6) qui, à l'état libre de forces externes et à une compensation de pression interne entre les deux chambres (5, 6), présente une plus petit volume que la chambre à compression (5), en ce que
a) la chambre à compression (5) en cas d'utilisation se trouve entièrement sous le talon ;
b) la chambre à expansion (6) présente une forme allongée avec une circonférence approximativement elliptique, et qui peut être disposée entre la cheville interne et le tendon d'Achille, en contact direct avec un système de veines perforantes local de sorte que, lorsque la chambre à compression (5) pouvant être disposée au niveau de la plante du pied est comprimée par la chambre à expansion (6) et pouvant alors être alors dilatée, le sang peut être expulsé hors d'une veine perforante dans cette région dans une veine profonde sous l'effet de la pression ;
c) une pièce intermédiaire tubulaire (7) reliant la chambre à compression (5) et la chambre à expansion (6) entre elles passe le long de la face interne du pied en cas d'utilisation ;
d) le dispositif comprend un collier de support (3) flexible mais pas élastique ou à peine extensible qui entoure de l'extérieur la chambre à expansion et à partir duquel la cheville peut être entourée de telle sorte
e) que la chambre à expansion (6) se gonflant lors de son remplissage depuis la chambre à compression (5) ne peut pas échapper vers l'extérieur, mais l'expansion de la chambre à expansion (6) est transmise au niveau de la veine perforante en dessous de la chambre à expansion (6), laquelle étant comprimée et vidée, et en ce que
f) le dispositif (1) ne recouvre pas le mollet en cas d'utilisation,
g) en outre en ce que la chambre à compression (5), la chambre à expansion (6) ainsi que la pièce intermédiaire tubulaire (7) sont réalisées par deux coupes superposées coïncidentes à partir d'un matériau compatible médicalement, lesquelles sont soudées les unes aux autres de manière étanche sur leur périmètre total,
h) et en ce que finalement une zone latérale libre (11) de la chambre à compression (5) est reliée au collier de support (3) par l'intermédiaire d'une courte bande de raccordement (4).

2. Dispositif (1) selon la revendication 1, **caractérisé en ce que** la longueur effective du collier de support (3) est réglable.

3. Dispositif (1) selon l'une des revendications précédentes, **caractérisé en ce que**, à l'état libre de forces externes et à compensation de pression interne entre les deux chambres (5, 6), la chambre à compression (5) présente le double volume de la chambre à expansion (6) ou plus, en particulier le triple volume de la chambre à expansion (6) ou plus.

4. Dispositif (1) selon l'une des revendications précédentes, **caractérisé en ce que** les deux chambres (5, 6) présentent une structure plane.

5. Dispositif (1) selon la revendication 4, **caractérisé en ce que**, à l'état libre de forces externes et à compensation de pression interne entre les deux chambres (5, 6), la chambre à compression (5) présente la double surface de base de la chambre à expansion (6) ou plus, en particulier la triple surface de base de la chambre à expansion (6) ou plus.

6. Dispositif (1) selon l'une des revendications précédentes, **caractérisé en ce qu'**il peut être porté dans une chaussure et/ou sous un bas.

7. Dispositif (1) selon l'une des revendications précédentes, **caractérisé en ce qu'**il est asymétrique.

8. Dispositif de prévention de la thrombose veineuse profonde des veines, **caractérisé par** une paire de dispositifs (1) présentant une symétrie miroir l'un par rapport à l'autre selon l'une des revendications précédentes pour le pied droit et le pied gauche (13) d'une personne.
